# EUROPÄISCHE PATENTSCHRIFT

(11) **EP 2 462 979 B1**
(45) Veröffentlichungstag und Bekanntmachung des Hinweises auf die Patenterteilung: **09.01.2013**
(21) Anmeldenummer: 11172089.2
(22) Anmeldetag: 30.06.2011
(51) Int. Cl.: A61M 37/00

(54) **Vorrichtung zum wiederholten Aufstechen von Haut und Stechmodul**
Device for repeated punctuation of skin and punctuation module
Dispositif de perçage répété de la peau et module de perçage

(30) Priorität: 10.12.2010 DE 202010013095 U
(43) Veröffentlichungstag der Anmeldung: 13.06.2012
(73) Patentinhaber: MT Derm GmbH, 14167 Berlin (DE)
(72) Erfinder: Jarling, Christian Reinhold, 12307 Berlin (DE)
(74) Vertreter: Bittner, Thomas L.

(56) Entgegenhaltungen:
- EP-A1- 1 958 659
- WO-A1-2007/015232
- DE-U1-202004 010 659
- KR-B1- 100 922 138

## Beschreibung

Die Erfindung betrifft eine Vorrichtung zum wiederholten Aufstechen von Haut sowie ein Stechmodul

### Hintergrund der Erfindung

Vorrichtungen zum wiederholten Aufstechen von Haut werden benutzt, um eine menschliche oder eine tierische Haut im Bereich der oberflächennahen Schichten lokal-aufzustechen. Hierdurch wird zum Beispiel die Voraussetzung geschaffen, in die Haut Wirkstoffe einzubringen, beispielsweise kosmetische oder medizinische Stoffe. In ähnlicher Weise wird das lokale Aufstechen von Haut genutzt, um einen Farbstoff zum Tätowieren oder zum Aufbringen von permanentem Make-up einzutragen.

Die Vorrichtungen zum wiederholten Aufstechen von Haut, die üblicherweise als Handgeräte ausgebildet werden, sind in Ausführungen bekannt, bei denen eine Antriebseinrichtung, zum Beispiel ein elektrischer Motor, genutzt wird, um eine Antriebskraft für das repetierende Aufstechen bereitzustellen. Die von der Antriebseinrichtung zur Verfügung gestellt Antriebskraft wird über einen Kopplungsmechanismus auf eine Stecheinrichtung gegeben, die zum lokalen Aufstechen der Haut vor und zurück bewegt wird. Als Stecheinrichtungen werden beispielsweise Einzelnadeln oder Nadelgruppen verwendet. Letzteres ist insbesondere bei Tätowiereinrichtungen der Fall.

Darüber hinaus wurde vorgeschlagen, sogenannte Nadelplatten für Stecheinrichtungen zu verwenden. Hierbei weist die Stecheinrichtung eine Nadelplatte auf, über deren Oberfläche mehrere Stechelemente beabstandet verteilt sind. Die Stechelemente sind beispielsweise als Nadel- oder Messerspitzen ausgeführt. In diesem Zusammenhang offenbart das Dokument DE 20 2004 010 659 U1 eine Vorrichtung zum Perforieren von Haut, bei der eine Nadelplatte mit Hilfe einer Antriebseinrichtung repetierend vor und zurück bewegt wird. Die Nadelplatte wird hierbei in einer Nadelplattenführungseinrichtung geführt. Die Nadelplattenführungseinrichtung dient dazu, ein definiertes Eindringen der Nadeln in die Haut vorzugeben, indem die Nadelplattenführungseinrichtung an den Seiten der Nadelplatte umlaufend eine Führung sicherstellt. Die Nadelplattenführungseinrichtung verhindert so ein Verkippen der Nadelplatte während der repetierenden Stechbewegung.

Aus dem Dokument US 2004 / 005882 A1 ist ebenfalls eine Vorrichtung zum Aufstechen von Haut bekannt, bei der Nadeln an einer Basis in Reihe angeordnet sind, wobei die Basis bei der repetierenden Stechbewegung an einem zugeordneten Gehäuseteil geführt ist.

WO 2007/015232 A1, KR 100922138 B1 und EP 1958659 A1 offenbaren weitere Vorrichtungen.

Ein Verfahren und eine Vorrichtung zum lokalen Aufstechen von Haut mittels einer Nadelplatte sind weiterhin aus dem Dokument US 2002 / 0123675 A1 bekannt.

Eine Vorrichtung zum lokalen Aufstechen von Haut ist weiterhin aus dem Dokument US 5,697,901 bekannt.

### Zusammenfassung der Erfindung

Aufgabe der Erfindung ist es, eine Vorrichtung zum wiederholten Aufstechen von Haut sowie ein Stechmodul zu schaffen, mit denen das lokale Hautaufstechen optimiert ist.

Diese Aufgabe wird erfindungsgemäß durch eine Vorrichtung zum wiederholten Aufstechen von Haut nach dem unabhängigen Anspruch 1 gelöst. Vorteilhafte Ausgestaltungen der Erfindung sind Gegenstand von abhängigen Unteransprüchen.

Die Erfindung umfasst den Gedanken einer Vorrichtung zum wiederholten Aufstechen von Haut mit einem Gehäuse, einer Antriebseinrichtung, die in dem Gehäuse aufgenommen und konfiguriert ist, eine Antriebskraft bereitzustellen, einem Kopplungsmechanismus, der in dem Gehäuse aufgenommen ist, und einer Stecheinrichtung, die über den Kopplungsmechanismus an die Antriebseinrichtung koppelt, derart, dass mittels des Kopplungsmechanismus die Antriebskraft für eine repetierende Stechbewegung auf die Stecheinrichtung gekoppelt wird, wobei die Stecheinrichtung eine Basis mit einer hieran gebildeten distalen Wirkfläche aufweist, über die mehrere beabstandete Stechelemente verteilt sind, und wobei die Basis mit der hieran gebildeten distalen Wirkfläche relativ zum Gehäuse verkippbar gelagert ist.

Weiterhin ist ein Stechmodul für eine solche Vorrichtung vorgesehen, welches ein Teilgehäuse und eine Stecheinrichtung aufweist, die an einen Teilkopplungsmechanismus koppelt, wobei die Stecheinrichtung eine Basis mit einer hieran gebildeten distalen Wirkfläche aufweist, über die mehrere beabstandete Stechelemente verteilt sind, und wobei die Basis mit der hieran gebildeten distalen Wirkfläche relativ zum Teilgehäuse verkippbar gelagert ist.

Bei der Erfindung ist vorgesehen, dass die Basis mit der hieran gebildeten distalen oder vorderseitigen Wirkfläche relativ zu dem Gehäuse verkippbar gelagert ist, was es ermöglicht, dass sich die Wirkfläche mit den hierauf gebildeten Stechelementen im Prozess des Aufstechens der Haut an die relative Lage der Hautoberfläche anpasst, insbesondere wenn das Gehäuse der Vorrichtung nicht senkrecht auf die Hautoberfläche aufgelegt ist. Im Stand der Technik verhindern die dort vorgesehenen Führungen der Nadelplatte gerade eine solche kippende Anpassung der Stellung der Nadelplatte. Für den Nutzer der Vorrichtung zum Aufstechen von Haut ist die Handhabbarkeit insgesamt erleichtert, da das lokale Aufstechen der Haut stets in optimierter Art und Weise stattfindet, auch wenn das Gehäuse der Vorrichtung und somit die geradlinige Bewegungsbahn des Hubes der Stecheinrichtung von dem Benutzer beim Führen der Vorrichtung über die Hautoberfläche ab und an verkippt werden, also nicht senkrecht zur Hautoberfläche angeordnet sind.

Für die auf der Wirkoberfläche angeordneten Stechelemente können verschiedenen Ausgestaltungen vorgesehen sein. So können Messer und / oder Nadeln oder auch Messer- und / oder Nadelspitzen gebildet sein. Nadeln oder Nadelspitzen können mit einer Kegelform gebildet sein. In einer Ausgestaltung kann vorgesehen sein, dass auf der distalen Wirkoberfläche die Stechelemente in einer oder mehreren Reihen angeordnet sind, die wahlweise umlaufen. Die Wirkfläche selbst kann hinsichtlich ihrer Grundform beliebigen Flächenformen entsprechen, beispielsweise kreisförmig, oval oder eckig sein. In einer Ausgestaltung kann vorgesehen sein, dass die Stechelemente im Wesentlichen einer Gleichverteilung entsprechend über die Wirkfläche verteilt sind. Alternativ kann vorgesehen sein, dass Stechelemente entlang des umlaufenden Randes der Wirkfläche verteilt sind, wohingegen ein mittlerer Bereich ganz frei von Stechelementen ist oder eine geringere Verteilungsdichte für die Stechelemente als im Randbereich aufweist.

Eine bevorzugte Weiterbildung der Erfindung sieht vor, dass die verkippbare Lagerung der Basis eine Kippbegrenzung aufweist. Die Kippbegrenzung kann in einer Ausführungsform mit einem ein- oder mehrseitigen Anschlag gebildet sein. In einer Ausführungsform ist vorgesehen, dass mit Hilfe der Kippbegrenzung das mögliche Verkippen der Basis mit der hieran gebildeten distalen Wirkfläche auf Kippwinkel von höchstens etwa 20°, bevorzugt höchstens etwa 10° und weiter bevorzugt höchstens etwa 5° begrenzt wird. Auch kann vorgesehen sein, dass Kippwinkel in verschiedenen Kipprichtungen unterschiedlich gebildet sind.

Bei einer zweckmäßigen Ausgestaltung der Erfindung kann vorgesehen sein, dass die verkippbare Lagerung der Basis mit einer Kugelkopflagerung gebildet ist. In dieser Ausführungsform oder in Verbindung mit anderen Lagerungsmechanismen kann vorgesehen sein, dass die Basis lösbar an den Kopplungsmechanismus koppelt. In einer Weiterbildung ist vorgesehen, dass auf diese Weise unterschiedlich ausgestaltete Basiselemente ankoppelbar sind, die sich zum Beispiel hinsichtlich der Form und / oder der Größe der Wirkfläche und / oder hinsichtlich der Stechelementegestaltung unterscheiden. Alternativ kann eine Kardangelenk-Kopplung vorgesehen sein.

Eine vorteilhafte Ausführungsform der Erfindung sieht vor, dass die Basis auch in einer verkippten Stellung frei von einem Kontakt mit dem Gehäuse gelagert ist. Bei dieser Ausführungsform ist die Basis also nicht nur in der nicht verkippten Stellung frei von Kontakt mit dem Gehäuse, sondern auch in den möglichen Kippstellungen.

Bevorzugt sieht eine Fortbildung der Erfindung vor, dass die distale Wirkfläche wenigstens in einer eingefahrenen Stellung hinter einer zugeordneten Gehäuseöffnung im Inneren des Gehäuses angeordnet ist. Dieses bedeutet, dass die Wirkfläche hinter der Fläche der zugeordneten Gehäuseöffnung angeordnet ist. Bevorzugt ist in einer Ausführungsform, dass auch die Stechelemente vollständig hinter die Fläche der zugeordneten Gehäuseöffnung zurücktreten in der eingefahrenen Stellung. Hierdurch wird beispielsweise die Verletzungsgefahr gemindert.

Bei einer vorteilhaften Ausgestaltung der Erfindung kann vorgesehen sein, dass die distale Wirkfläche in einer ausgefahrenen Stellung in der Ebene der Fläche der zugeordneten Gehäuseöffnung angeordnet ist. Das Anordnen der distalen Wirkfläche in der Fläche der zugeordneten Gehäuseöffnung bedeutet, dass die beabstandeten Stechelemente von der Fläche der zugeordneten Gehäuseöffnung nach außen vorstehen. Ist die distale Wirkfläche in der ausgefahrenen Stellung der Basis verkippt, bedeutet dies, dass die Fläche der zugeordneten Gehäuseöffnung und die distale Wirkfläche zueinander verkippt sind. Alternativ kann vorgesehen sein, dass in der ausgefahrenen Stellung die distale Wirkfläche noch hinter der Fläche der zugeordneten Gehäuseöffnung oder dieser vorgelagert angeordnet ist.

Eine Weiterbildung der Erfindung kann vorsehen, dass die distale Wirkfläche sich beim Verlagern zwischen der eingefahrenen und der ausgefahrenen Stellung im Rahmen der repetierenden Stechbewegung überwiegend in einem umschlossenen Gehäusebereich bewegt. Bei dieser Ausgestaltung bewegt sich die Basis auf mehr als 50% der Hublänge der repetierenden Stechbewegung in einem Bereich, der von dem Gehäuse umschlossen ist.

Eine bevorzugte Weiterbildung der Erfindung sieht vor, dass im Bereich der distalen Wirkfläche Durchgangsöffnungen angeordnet sind, die in die Stechelemente integriert und / oder getrennt hiervon gebildet sind. Die Durchgangsöffnungen ermöglichen es in einer Ausgestaltung, dass ein in die Hautoberfläche einzubringender Wirkstoff zu den Aufstechstellen zugeführt wird, weshalb es sich dann um Zuführöffnungen handelt. Alternativ oder ergänzend können die Durchgangsöffnungen wenigstens teilweise als Abführöffnungen ausgeführt sein, mit denen Flüssigkeit von dem Bereich zwischen Hautoberfläche und Wirkfläche nach hinten abgeführt wird. Auf diese Weise wird zum Beispiel verhindert, dass sich zwischen der Wirkfläche und der Hautoberfläche ein Flüssigkeitsfilm bildet, der den Aufstechprozess verhindert oder behindert. Auch eine Ausbildung von Zuführ- / Abführöffnungen kann vorgesehen sein, also von Durchgangsöffnungen, die eine kombinierte Funktion erfüllen. Die Durchgangsöffnungen können beispielsweise in den Stechelementen selbst gebildet sein, indem diese zum Beispiel als Hohlnadeln ausgeführt sind. Alternativ oder ergänzend können getrennt von den Stechelementen Durchgangsöffnungen in der Basis vorgesehen sein. In einer Ausführungsform weisen die Durchgangsöffnungen einen Durchmesser von etwa 0,3 bis etwa 0,5mm, vorzugsweise von etwa 0,4mm auf.

Bei einer zweckmäßigen Ausgestaltung der Erfindung kann vorgesehen sein, dass die distale Wirkfläche konvex gekrümmt ist. Die konvexe Form der distalen Wirkfläche kann eine zwei- oder dreidimensionale Ausgestaltung aufweisen, wobei letzteres in Form eines Kugeloberflächenabschnitts ausgeführt sein kann.

Eine vorteilhafte Ausführungsform der Erfindung sieht vor, dass die Basis an ein distales Ende eines von dem Kopplungsmechanismus umfassten Schaftelementes koppelt, wobei das Schaftelement in einer Schaftführung läuft. Die Schaftführung ist beispielsweise mit Hilfe einer Buchse ausgeführt. In einer Weiterbildung kann vorgesehen sein, dass das Schaftelement verdrehsicher in der Schaftführung aufgenommen ist. Beispielsweise ist eine mechanische Verdrehsicherung vorgesehen. Alternativ oder ergänzend kann mittels einer magnetischen Verdrehsicherung ein unbeabsichtigtes Verdrehen des Schaftelementes bei der repetierenden Stechbewegung verhindert sein.

Bevorzugt sieht eine Fortbildung der Erfindung vor, dass ein proximales Ende des Schaftelementes durch eine Membranöffnung geführt ist. Mit Hilfe der Membran aus elastischem Material ist ein vorderer Gehäuseabschnitt abgedichtet, so dass insbesondere Flüssigkeitsreste nicht zur Antriebseinrichtung gelangen können, auch wenn das Schaftelement vor und zurück bewegt wird. Bei einer Ausführungsform ist vorgesehen, dass mit Hilfe der Membran, die zum Beispiel U-förmig oder topfförmig ausgeführt sein kann, eine Rückstell- oder Rückholkraft für das Schaftelement und somit die Stecheinrichtung bereitgestellt ist. Diese Rückstellkraft kann ergänzend oder für sich allein die Rückstellung der Stecheinrichtung in die eingefahrene Stellung bewirken. Ist die Vorrichtung modulartig aufgebaut, so kann die Membran aus dem elastischen Material ein Stechmodul rückseitig abdichten.

Bei einer vorteilhaften Ausgestaltung der Erfindung kann vorgesehen sein, dass auf dem Schaftelement und dieses umgreifend ein Zusatzelement angeordnet ist. Das Zusatzelement ist in einer Ausführungsform zwischen der Schaftführung und der elastischen Membran auf dem Schaftelement angeordnet. Bei dieser Ausgestaltung kann vorgesehen sein, dass die Schaftführung rückseitig, also auf einer dem Zusatzelement zugewandten Seite, eine Aufnahme für das Zusatzelement aufweist. An dem Schaftelement ist bei einer Weiterbildung ein Vorsprung vorgesehen, welcher ein unbeabsichtigtes Verrutschen des Zusatzelementes auf dem Schaftelement verhindert. Das Zusatzelement kann als Abstreif- und / oder Dämpfungselement ausgeführt sein.

Eine Weiterbildung der Erfindung kann vorsehen, dass ein vorderer Gehäuseabschnitt lösbar an dem Gehäuse angeordnet ist. In einer Ausführungsform ist in dem vorderen Gehäuseabschnitt die Basis der Stecheinrichtung aufgenommen. Es kann vorgesehen sein, dass eine innere Oberfläche im vorderen Gehäuseabschnitt konkav ausgeführt ist. Eine solche Ausgestaltung ist sowohl bei der Ausführung mit lösbarem Gehäuseabschnitt als bei nicht lösbarem Gehäuseabschnitt möglich.

Bei einer zweckmäßigen Ausgestaltung der Erfindung kann vorgesehen sein, dass das Gehäuse aus mehreren Modulen mit einem jeweils zugeordneten Teilgehäuse gebildet ist, die lösbar miteinander verbunden sind, wobei die mehreren Module ein Antriebsmodul mit der Antriebseinrichtung und ein Stechmodul umfassen, in welchem wenigstens das Schaftelement und die hieran koppelnde Basis aufgenommen sind. Es kann vorgesehen sein, dass das Stechmodul als ein steriles Einwegmodul ausgeführt ist.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Schnittdarstellung einer Vorrichtung zum wiederholten Aufstechen von Haut mit einem Antriebsmodul und einem hieran koppelnden Stechmodul teilweise im Aufriss,
- Fig. 2: eine schematische Schnittdarstellung des Stechmoduls für die Vorrichtung nach Fig. 1,
- Fig. 3: eine perspektivische Darstellung des Stechmoduls aus Fig. 2 im Schnitt,
- Fig. 4: schematische Darstellungen einer Basis einer Stecheinrichtung und
- Fig. 5: schematische Darstellungen einer Schaftelementführung.

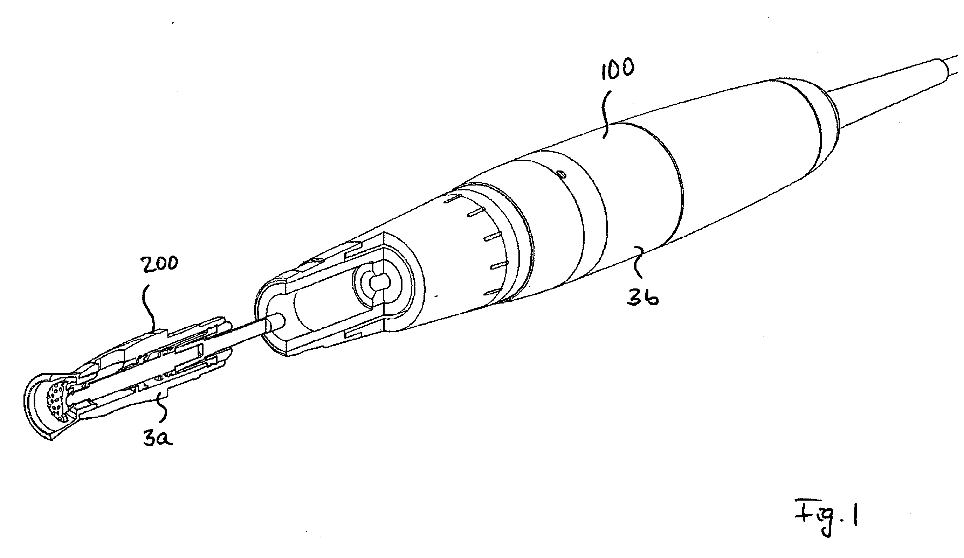

Fig. 1 zeigt eine schematische Schnittdarstellung einer Vorrichtung zum wiederholten Aufstechen von Haut mit einem Antriebsmodul 100 und einem hieran koppelnden Stechmodul 200. Bei der gezeigten Darstellung ist das Stechmodul 200 von dem Antriebsmodul 100 gelöst.

Bei der üblicherweise als Handgerät ausgeführten Vorrichtung zum Aufstechen von Haut koppelt das Stechmodul 200 endseitig an das Antriebsmodul 100, welches eine Antriebseinrichtung zum Bereitstellen einer Antriebskraft umfasst, beispielsweise einen Elektromotor. Derartige Antriebsmodule sind in verschiedenen Ausführungsformen als solche bekannt und werden hier deshalb nicht weiter erläutert.

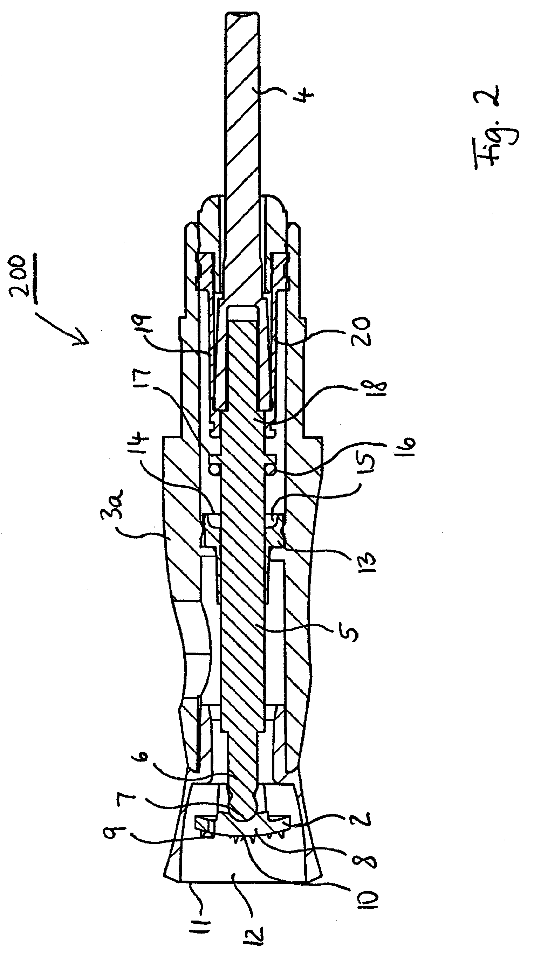

Fig. 2 zeigt eine Schnittdarstellung des Stechmoduls 200. Ein Kopplungsmechanismus, welcher teilweise in dem Antriebsmodul 100 und teilweise in dem Stechmodul 200 gebildet ist, dient zur Einleitung der von der Antriebseinrichtung bereitgestellten Antriebskraft in eine Stecheinrichtung 2, so dass diese eine repetierende Vor- und Rückwärtsbewegung ausführt. Fig. 2 zeigt hierbei die Stecheinrichtung 2 in einer eingefahrenen Stellung.

Die Stecheinrichtung 2 ist bei den Darstellungen in den Fig. 1 und 2 in einem dem Stechmodul 200 zugeordneten Gehäuseteil 3a angeordnet, welches zusammen mit einem dem Antriebsmodul 100 zugeordneten Gehäuseteil 3b ein Gehäuse 3 der Vorrichtung bildet.

Für den Kopplungsmechanismus sind in Fig. 2 eine Verlängerung 4 sowie ein Schaftelement 5 gezeigt, welches in die Verlängerung 4 eingesteckt ist. Am distalen Ende 6 des Schaftelementes 5 ist mit Hilfe einer Kugelkopflagerung 7 die Stecheinrichtung 2 an das Schaftelement 5 gekoppelt. Die Kugelkopflagerung 7 ermöglicht, dass eine Basis 8 der Stecheinrichtung 2 verschiedene Kippstellungen einnehmen kann, sei es in der in Fig. 2 dargestellten eingefahrenen Stellung oder in einer ausgefahrenen Stellung (nicht dargestellt), in welcher wenigstens Stechelemente 9, die auf einer Wirkfläche 10 der Basis 8 der Stecheinrichtung 2 angeordnet sind, vor einer Fläche 11 einer der Stecheinrichtung 2 zugeordneten Gehäuseöffnung 12 angeordnet sind, so dass die menschliche oder tierische Haut aufgestochen werden kann. Die hinsichtlich des Schaftelementes 5 distal an der Basis 8 angeordnete Wirkfläche 10 ist konvex ausgeführt. Im Bereich hinter der Gehäuseöffnung 12 ist die innere Gehäuseoberfläche gegenüber der Basis 8 konkav, was eine freie Verkippbarkeit der Basis 8 an der Kugelkopflagerung 7 unterstützt.

Bei der repetierenden Vor- und Rückwärtsbewegung gleitet das Schaftelement 5 in einer als Buchse ausgeführten Schaftführung 13, mit der das Schaftelement 5 zusätzlich auch gegen ein unbeabsichtigtes Verdrehen um seine Längsachse gesichert ist. Insoweit ist bei dieser Ausführungsform auch eine Verdrehsicherung gebildet mittels der Schaftführung 13. Auf einer Rückseite 14 weist die Schaftführung 13 eine umlaufende Aufnahme 15 für ein als Abstreif- / Dämpfungselement ausgeführtes Zusatzelement 16 auf, welches im dargestellten Ausführungsbeispiel als ein O-Ring gebildet ist. Das Zusatzelement 16 lagert bei der Darstellung in Fig. 2 an einem Vorsprung 17, welcher rückseitig den Bereich des Schaftelementes 5 begrenzt, in den das Zusatzelement 16 gelangen kann.

Ein proximales Ende 18 des Schaftelementes 5 wird von einer Membran 19 aus elastischem Material umgeben. Mit Hilfe der Membran 19 erfolgt eine Abdichtung des der Membran 19 vorgelagerten Raumes in dem Gehäuseteil 3a des Stechmoduls 200. Wird die Stecheinrichtung 2 mit Hilfe der Antriebskraft in die ausgefahrene Stellung verlagert, so werden seitliche Abschnitte 20 der Membran 19 gestreckt, wodurch eine Rückstell- oder Rückholkraft auf die Verlängerung 4 und das Schaftelement 5 ausgeübt wird.

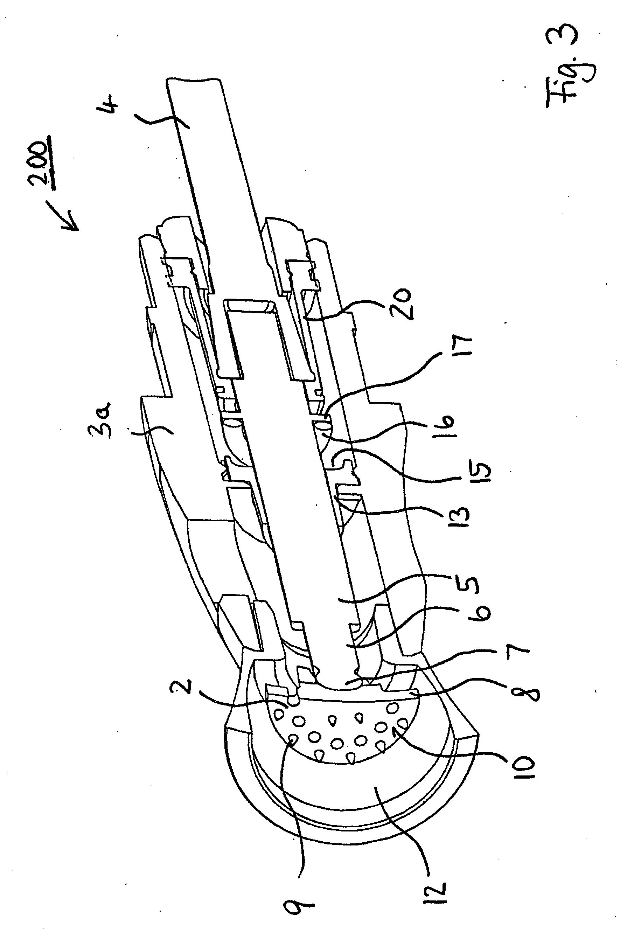

Fig. 3 zeigt das Stechmodul 200 aus Fig. 2 perspektivisch im Schnitt.

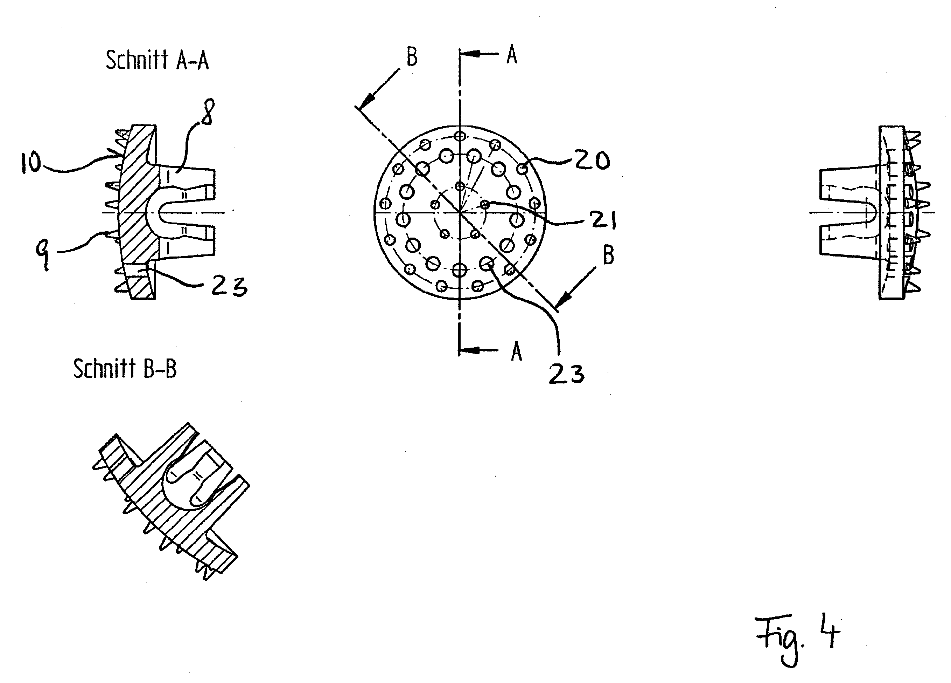

Fig. 4 zeigt schematische Darstellungen der Basis 8 der Stecheinrichtung 2. Die Stechelemente 9, bei denen es sich um Messer- oder Nadelspitzen handeln kann, sind entlang eines Außenrings 21 und eines Innenrings 22 jeweils gleich verteilt angeordnet, also mit gleichen Abständen zu den beidseitigen Nachbarn. Zwischen dem Außenring 21 und dem Innenring 22 sind Durchgangsöffnungen 23 gebildet.

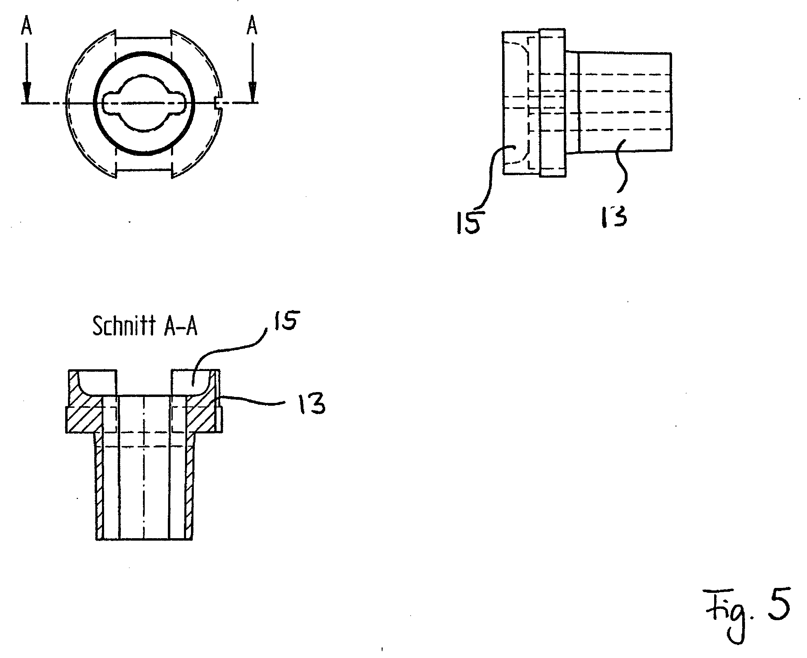

Fig. 5 zeigt schematische Darstellungen der Schaftführung 13.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungen von Bedeutung sein.

## Patentansprüche

1. Vorrichtung zum wiederholten Aufstechen von Haut, mit:
- einem Gehäuse (3),
- einer Antriebseinrichtung, die in dem Gehäuse (3) aufgenommen und konfiguriert ist, eine Antriebskraft bereitzustellen,
- einem Kopplungsmechanismus, der in dem Gehäuse (3) aufgenommen ist, und
- einer Stecheinrichtung (2), die über den Kopplungsmechanismus an die Antriebseinrichtung koppelt, derart, dass mittels des Kopplungsmechanismus die Antriebskraft für eine repetierende Stechbewegung auf die Stecheinrichtung (2) gekoppelt wird,
wobei die Stecheinrichtung (2) eine Basis (8) mit einer hieran gebildeten distalen Wirkfläche (10) aufweist, über die mehrere beabstandete Stechelemente (9) verteilt sind, und wobei die Basis (8) mit der hieran gebildeten distalen Wirkfläche (10) relativ zum Gehäuse (3) verkippbar gelagert ist.

2. Vorrichtung nach Anspruch 1, **dadurch gekennzeichnet, dass** die verkippbare Lagerung der Basis (8) eine Kippbegrenzung aufweist.

3. Vorrichtung nach Anspruch 1 oder 2, **dadurch gekennzeichnet, dass** die verkippbare Lagerung der Basis mit einer Kugelkopflagerung (7) gebildet ist.

4. Vorrichtung nach mindestens einem der vorangehenden Ansprüche, **dadurch gekennzeichnet, dass** die Basis (8) auch in einer verkippten Stellung frei von einem Kontakt mit dem Gehäuse (3) gelagert ist.

5. Vorrichtung nach mindestens einem der vorangehenden Ansprüche, **dadurch gekennzeichnet, dass** die distale Wirkfläche (10) wenigstens in einer eingefahrenen Stellung hinter einer zugeordneten Gehäuseöffnung (12) im Inneren des Gehäuses (3) angeordnet ist.

6. Vorrichtung nach Anspruch 5, **dadurch gekennzeichnet, dass** die distale Wirkfläche (10) in einer ausgefahrenen Stellung in der Ebene der Fläche (11) der zugeordneten Gehäuseöffnung (12) angeordnet ist.

7. Vorrichtung nach Anspruch 5 oder 6, **dadurch gekennzeichnet, dass** die distale Wirkfläche (10) sich beim Verlagern zwischen der eingefahrenen und der ausgefahrenen Stellung im Rahmen der repetierenden Stechbewegung überwiegend in einem umschlossenen Gehäusebereich bewegt.

8. Vorrichtung nach mindestens einem der vorangehenden Ansprüche, **dadurch gekennzeichnet, dass** im Bereich der distalen Wirkfläche (10) Durchgangsöffnungen (23) angeordnet sind, die in die Stechelemente (9) integriert und / oder getrennt hiervon gebildet sind.

9. Vorrichtung nach mindestens einem der vorangehenden Ansprüche, **dadurch gekennzeichnet, dass** die distale Wirkfläche (10) konvex gekrümmt ist.

10. Vorrichtung nach mindestens einem der vorangehenden Ansprüche, **dadurch gekennzeichnet, dass** die Basis (8) an ein distales Ende (6) eines von dem Kopplungsmechanismus umfassten Schaftelementes (5) koppelt, wobei das Schaftelement (5) in einer Schaftführung (13) läuft.

11. Vorrichtung nach Anspruch 10, **dadurch gekennzeichnet, dass** ein proximales Ende des Schaftelementes (5) durch eine Membranöffnung geführt ist.

12. Vorrichtung nach Anspruch 10 oder 11, **dadurch gekennzeichnet, dass** auf dem Schaftelement (5) und dieses umgreifend ein Zusatzelement (16) angeordnet ist.

13. Vorrichtung nach mindestens einem der vorangehenden Ansprüche, **dadurch gekennzeichnet, dass** ein vorderer Gehäuseabschnitt lösbar an dem Gehäuse (3) angeordnet ist.

14. Vorrichtung nach mindestens einem der vorangehenden Ansprüche, soweit auf Anspruch 10 rückbezogen, **dadurch gekennzeichnet, dass** das Gehäuse (3) aus mehreren Modulen mit einem jeweils zugeordneten Teilgehäuse gebildet ist, die lösbar miteinander verbunden sind, wobei die mehreren Module ein Antriebsmodul mit der Antriebseinrichtung und ein Stechmodul (1) umfassen, in welchem wenigstens das Schaftelement (5) und die hieran koppelnde Stecheinrichtung (2) aufgenommen sind.

15. Stechmodul (1) für eine Vorrichtung zum wiederholten Aufstechen von Haut nach mindestens einem der vorangehenden Ansprüche, mit:
- einem Teilgehäuse (3a) und
- einer Stecheinrichtung (2), die an einen Teilkopplungsmechanismus koppelt,
wobei die Stecheinrichtung (2) eine Basis (8) mit einer hieran gebildeten distalen Wirkfläche (10) aufweist, über die mehrere beabstandete Stechelemente (9) verteilt sind, und wobei die Basis (8) mit der hieran gebildeten distalen Wirkfläche (10) relativ zum Teilgehäuse verkippbar gelagert ist.

## Claims

1. A device for repeated piercing of skin, having:
- a housing (3);
- a drive device which is accommodated in the housing (3) and configured to provide power;
- a coupling mechanism which is accommodated in the housing (3); and
- a piercing device (2) which couples to the drive device via the coupling mechanism in a manner such that the power for a repetitive piercing movement is coupled to the piercing device (2) by means of the coupling mechanism;
wherein the piercing device (2) has a base (8) having a distal working head (10) formed thereon over which a plurality of separated piercing elements (9) are distributed, and wherein the base (8) with the distal working head (10) formed thereon is tiltably mounted with respect to the housing (3).

2. The device as claimed in claim 1, **characterized in that** the tiltable mount for the base (8) has a tilt limiter.

3. The device as claimed in claim 1 or claim 2, **characterized in that** the tiltable mount for the base is formed by a ball joint mount (7).

4. The device as claimed in at least one of the preceding claims, **characterized in that** the base (8) is mounted such that even in a tilted position, it does not come into contact with the housing (3).

5. The device as claimed in at least one of the preceding claims, **characterized in that** at least in a retracted position, the distal working head (10) is arranged inside the housing (3) behind an associated housing opening (12).

6. The device as claimed in claim 5, **characterized in that** in the deployed position, the distal working head (10) is arranged in the plane of the surface (11) of the associated housing opening (12).

7. The device as claimed in claim 5 or claim 6, **characterized in that** upon displacement between the retracted and deployed position in the context of a repetitive piercing movement, the distal working head (10) moves substantially in an enclosed region of the housing.

8. The device as claimed in at least one of the preceding claims, **characterized in that** in the region of the distal working head (10), through-openings (23) are arranged which are integrated into the piercing elements (9) and/or are formed separately thereof.

9. The device as claimed in at least one of the preceding claims, **characterized in that** the distal working head (10) is convexly curved.

10. The device as claimed in at least one of the preceding claims, **characterized in that** the base (8) is coupled to a distal end (6) of a shaft element (5) comprised in the coupling mechanism, wherein the shaft element (5) runs in a shaft guide (13).

11. The device as claimed in claim 10, **characterized in that** a proximal end of the shaft element (5) is guided through a membrane opening.

12. The device as claimed in claim 10 or claim 11, **characterized in that** an additional element (16) is arranged on the shaft element (5), encompassing it.

13. The device as claimed in at least one of the preceding claims, **characterized in that** a frontal housing section is detachably arranged on the housing (3).

14. The device as claimed in at least one of the preceding claims insofar as they depend on claim 10, **characterized in that** the housing (3) is formed from a plurality of modules each with an associated housing portion which are detachably connected together, wherein the plurality of modules comprise a drive module with a drive device and a piercing module (1), in which at least the shaft element (5) and the piercing device (2) coupled thereto are accommodated.

15. A piercing module (1) for a device for repeated piercing of skin according to at least one of the preceding claims, having:
- a housing portion (3a); and
- a piercing device (2) which couples to a coupling mechanism portion;
wherein the piercing device (2) has a base (8) with a distal working head (10) formed thereon, over which a plurality of separated piercing elements (9) are distributed, and wherein the base (8) with the distal working head (10) formed is tiltably mounted thereon with respect to the housing portion.

## Revendications

1. Dispositif de perçage répété de la peau, comprenant :
- un boîtier (3),
- un dispositif d'entraînement reçu dans le boîtier (3) et configuré pour fournir une force d'entraînement,
- un mécanisme de couplage reçu dans le boîtier (3), et
- un dispositif de perçage (2) couplé au dispositif d'entraînement par le mécanisme de couplage, de telle sorte que le mécanisme de couplage permet de coupler la force d'entraînement pour un mouvement de perçage répété avec le dispositif de perçage (2),
dans lequel le dispositif de perçage (2) présente une base (8) avec une surface active (10) distale formée sur celle-ci et sur laquelle sont répartis plusieurs éléments de perçage (9) espacés, et dans lequel la base (8) avec la surface active (10) distale formée sur celle-ci est logée de manière pivotante par rapport au boîtier (3).

2. Dispositif selon la revendication 1, **caractérisé en ce que** le logement pivotant de la base (8) présente une limitation de pivotement.

3. Dispositif selon la revendication 1 ou 2, **caractérisé en ce que** le logement pivotant de la base est formé par un logement à tête sphérique (7).

4. Dispositif selon au moins l'une des revendications précédentes, **caractérisé en ce que** la base (8) est logée sans contact avec le boîtier (3), même dans une position de pivotement.

5. Dispositif selon au moins l'une des revendications précédentes, **caractérisé en ce que** la surface active distale (10) est disposée l'intérieur du boîtier (3) au moins dans une position rentrée derrière une ouverture de boîtier (12) associée.

6. Dispositif selon la revendication 5, **caractérisé en ce que** la surface active distale (10) est disposée dans une position sortie dans le plan de la surface (11) de l'ouverture de boîtier (12) associée.

7. Dispositif selon la revendication 5 ou 6, **caractérisé en ce que** lors d'une translation entre la position rentrée et la position sortie, la surface active distale (10) se déplace principalement dans une zone de boîtier confinée dans le cadre du mouvement de perçage répété.

8. Dispositif selon au moins l'une des revendications précédentes, **caractérisé en ce que** dans la zone de la surface active distale (10), des ouvertures de passage (23) sont disposées qui sont intégrées dans les éléments de perçage (9) et/ou formées séparément de ceux-ci.

9. Dispositif selon au moins l'une des revendications précédentes, **caractérisé en ce que** la surface active distale (10) présente une courbure convexe.

10. Dispositif selon au moins l'une des revendications précédentes, **caractérisé en ce que** la base (8) est couplée à une extrémité distale (6) d'un élément de tige (5) enserré par le mécanisme de couplage, dans lequel l'élément de tige (5) s'étend dans un guidage de tige (13).

11. Dispositif selon la revendication 10, **caractérisé en ce qu'**une extrémité proximale de l'élément de tige (5) est guidée à travers une ouverture à membrane.

12. Dispositif selon la revendication 10 ou 11, **caractérisé en ce que** sur l'élément de tige (5), un élément supplémentaire (16) est disposé en entourant celui-ci.

13. Dispositif selon au moins l'une des revendications précédentes, **caractérisé en ce qu'**une partie de boîtier avant est disposée de manière amovible sur le boîtier (3).

14. Dispositif selon au moins l'une des revendications précédentes, dans la mesure où elle se réfère à la revendication 10, **caractérisé en ce que** le boîtier (3) est formé de plusieurs modules avec un boîtier partiel associé respectivement et qui sont reliés de manière amovible, dans lequel les plusieurs modules comprennent un module d'entraînement avec un dispositif d'entraînement et un module de perçage (1) dans lequel sont reçus au moins l'élément de tige (5) et le dispositif de perçage (2) couplé à celui-ci.

15. Module de perçage (1) pour un dispositif de perçage répété de la peau selon au moins l'une des revendications précédentes, comprenant :
- un boîtier partiel (3 a), et
- un dispositif de perçage (2) couplé à un mécanisme de couplage partiel,
dans lequel le dispositif de perçage (2) présente une base (8) avec une surface active distale (10) formée sur celle-ci et sur laquelle sont répartis plusieurs éléments de perçage (9) espacés, et dans lequel la base (8) avec la surface active distale (10) formée sur celle-ci est logée de manière pivotantepar rapport au boîtier partiel.
